# EUROPÄISCHE PATENTSCHRIFT

(11) **EP 1 219 256 B1**
(45) Veröffentlichungstag und Bekanntmachung des Hinweises auf die Patenterteilung: **05.11.2003**
(21) Anmeldenummer: 01130460.7
(22) Anmeldetag: 20.12.2001
(51) Int. Cl.: A61B 17/70

(54) **Fixierelement**
Fixing element
Elément de fixation

(30) Priorität: 22.12.2000 DE 10064571
(43) Veröffentlichungstag der Anmeldung: 03.07.2002
(73) Patentinhaber: Biedermann, Lutz, 78048 Villingen-Schwenningen (DE); Harms, Jürgen, Prof. Dr., 76227 Karlsruhe (DE)
(72) Erfinder: Biedermann, Lutz, 78048 Villingen-Schwenningen (DE); Harms, Jürgen, Prof. Dr., 76227 Karlsruhe (DE)
(74) Vertreter: Prüfer, Lutz H., Dipl.-Phys.

(56) Entgegenhaltungen:
- EP-A- 0 614 649
- WO-A-01/08574
- WO-A-98/32386
- US-A- 5 360 431
- US-A- 5 630 817

## Beschreibung

Die Erfindung betrifft ein Fixierelement nach dem Oberbegriff des Patentanspruches 1.

Eine derartiges Fixierelement ist in Form einer Knochenschraube aus der EP 0 614 649 A1 bekannt. Bei dieser ist zum perfekten Verriegeln der Stab-Knochenschrauben-Verbindung eine in die offene Bohrung einzuschraubende Sicherungsmutter vorgesehen.

Aufgabe der Erfindung ist es, ein Fixierelement der eingangs beschriebenen Art zu schaffen, die ohne eine solche Innenmutter auskommt.

Diese Aufgabe wird durch die in Patentanspruch 1 gekennzeichnete Knochenschraube gelöst.

Weiterbildungen der Erfindung sind in den Unteransprüchen gekennzeichnet.

Die Erfindung wird nun in der Beschreibung eines Ausführungsbeispieles anhand der Figuren näher erläutert.

Von den Figuren zeigen:
- Fig. 1: eine explosionsartige Seitenansicht der Knochenschraube;
- Fig. 2: eine Schnittdarstellung durch die Knochenschraube;
- Fig. 3: eine Explosionsdarstellung der Außenmutter in vergrößertem Maßstab, teilweise geschnitten;
- Fig. 4: die Außenmutter in zusammengesetztem Zustand vor der Einwirkung auf einen Stab; und
- Fig. 5: die selbe Darstellung nach der Einwirkung auf den Stab;
- Fig. 6: eine abgewandelte Ausführungsform.

Die als Ausführungsbeispiel gezeigte Knochenschraube weist das eigentliche Schraubenelement 1 mit einem Gewindeabschnitt 2 und einem Kopf 3 auf. Der Kopf ist angrenzend an den Gewindeabschnitt kugelsegmentförmig ausgebildet. Koaxial zur Gewindeachse und auf dem dem Gewindeabschnitt 2 gegenüberliegenden Ende weist der Kopf eine Ausnehmung 4 zum Ineingriffbringen mit einem Inbusschlüssel auf.

Die Knochenschraube umfaßt ferner ein zylindrisch ausgebildetes Aufnahmeteil 5. Dieses weist an seinem einen Ende eine axialsymmetrisch ausgerichtete erste Bohrung 7 auf, deren Durchmesser größer als der des Gewindeabschnittes 2 und kleiner als der des Kopfes 3 ist. Das Aufnahmeteil 5 weist ferner eine koaxiale zweite Bohrung 8 auf, die auf dem der ersten Bohrung 7 gegenüberliegenden Ende offen ist und deren Durchmesser so groß ist, daß das Schraubenelement 1 durch das offene Ende mit seinem Gewindeabschnitt 2 durch die erste Bohrung 7 hindurch und mit dem Kopf 3 bis zum Grund der zweiten Bohrung führbar ist. Zwischen der ersten und der zweiten Bohrung ist ein kleiner koaxialer Abschnitt 9 vorgesehen, der unmittelbar an die erste Bohrung angrenzt und zum offenen Bereich hin sphärisch ausgebildet ist, wobei der Radius im wesentlichen gleich dem Radius des kugelsegmentförmigen Abschnittes des Kopfes 3 ist. Ferner weist das Aufnahmeteil 5 eine zur Mitte des Teiles symmetrisch angeordnete U-förmige Ausnehmung 6 auf, deren Grund zu der ersten Bohrung 7 hin gerichtet ist und deren beide Seitenschenkel 30, 31 sich zu dem der ersten Bohrung 7 abgewandten offenen Ende hin erstrecken. Am freien Ende der Schenkel der U-förmigen Ausnehmung ist ein Außengewinde 11 vorgesehen.

Auf der am freien Ende des Kopfes 3 liegenden Seite befindet sich eine Druckscheibe 11, die so ausgebildet ist, daß sie auf ihrer dem Kopf 3 zugewandten Seite eine sphärische Absenkung aufweist, deren Radius im wesentlichen gleich dem Radius des kugelsegmentförmigen Abschnittes des Kopfes ist. Der Außendurchmesser der Druckscheibe 11 ist so gewählt, daß diese in den Zylinderabschnitt 12 der zweiten Bohrung 8 eine Gleitbewegung ausführen kann, also in dem Zylinderabschnitt zu dem Kopf hin verschiebbar ist. Die Druckscheibe weist eine koaxiale Bohrung auf, die einen Zugriff zur Ausnehmung 4 ermöglicht.

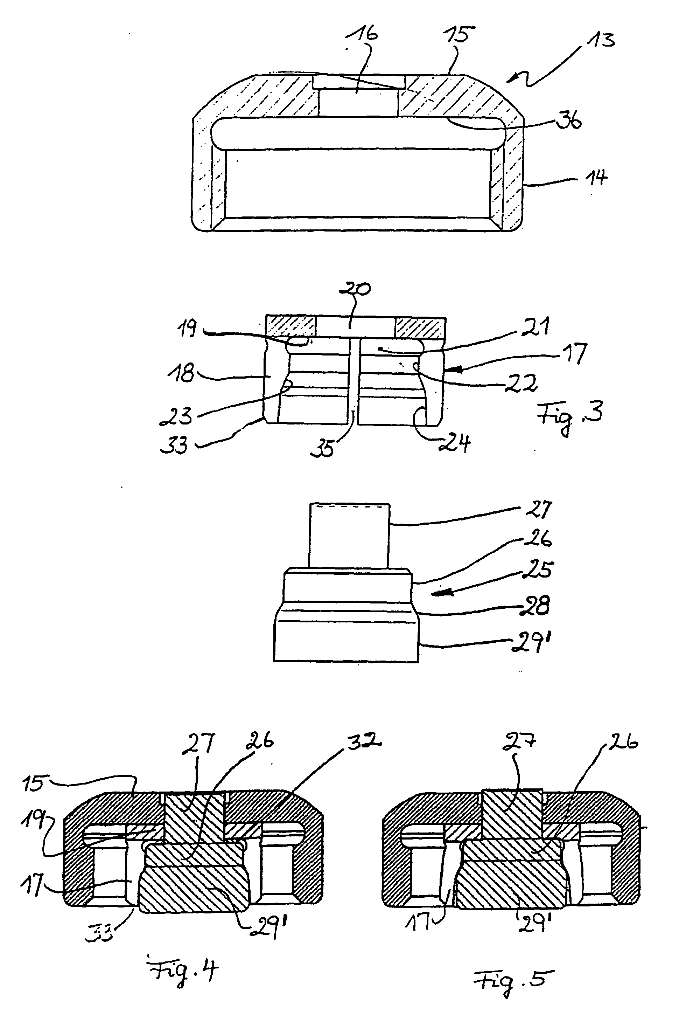

Die Knochenschraube umfaßt ferner eine Außenmutter 13, die im weiteren im Detail insbesondere anhand der Figuren 3 bis 5 erläutert wird. Die Außenmutter ist als eine Hutmutter ausgebildet und weist einen zylindrischen seitlichen Rand mit einem Innengewindeabschnitt 14, der die eigentliche Mutter bildet, und einen an dem einen Rand des Gewindeabschnittes 14 anschließenden Deckelteil 15 auf. Der Deckelteil ist wie bei einer üblichen Hutmutter ausgebildet, weist aber zusätzlich eine konzentrische Bohrung 16 mit einem ersten Durchmesser auf. Es ist ferner eine Hülse 17 vorgesehen, die einen Mantel 18 und auf ihrer dem Deckelteil 15 zugewandten Seite einen Boden 19 aufweist. In dem Boden 19 ist eine konzentrische Bohrung 20 mit einem zweiten Durchmesser vorgesehen. Der zweite Durchmesser ist gleich dem ersten Durchmesser der Bohrung 16 oder ein wenig größer.

Wie am besten aus Fig. 3 ersichtlich ist, weist der Mantel 18 einen an den Boden 19 angrenzenden Innenraum 21 auf, der einen zylindrischen Wandabschnitt 22 umfaßt, welcher in einem vorbestimmten Abstand von dem Boden endet. In dem gezeigten Ausführungsbeispiel liegt zwischen dem zylindrischen Wandabschnitt 22 und dem Boden eine Hinterschneidung, deren Durchmesser wenigstens gleich dem Durchmesser des zylindrischen Wandabschnittes 22 ist. Grundsätzlich kann sich der zylindrische Wandabschnitt auch bis zum Boden 19 selbst hin erstrecken. Wie aus Fig. 3 ersichtlich ist, schließt sich im Inneren des Mantels an den dem Boden 19 abgewandten Rand des zylindrischen Wandabschnittes 22 ein nach außen konvergent verlaufender Wandabschnitt 23 an, der nach außen so abgeschrägt ist, daß er die Form eines Kegelabschnittes aufweist, wobei der Neigungswinkel der Schräge gegenüber der Zylinderinnenwand etwa 30° bis 60° und vorzugsweise etwa 40 ° beträgt. Der Außendurchmesser der außen zylindrischen Hülse 17 ist nahezu gleich dem Durchmesser der zweiten Bohrung 8 und um soviel kleiner als letztere, daß die Hülse gerade ohne Reibung in die zweite Bohrung 8 einführbar ist.

Wie ferner am besten aus Fig. 3 ersichtlich ist, schließt sich an den nach außen größer werdenden kegelstumpfförmigen Wandabschnitt 23 ein weiterer Wandabschnitt 24 an, der zwar nach außen noch im Durchmesser zunimmt, also wiederum als Kegelabschnitt ausgebildet ist, dessen Neigung jedoch nur wenige Grade beträgt. Alternativ kann dieser Wandabschnitt 24 auch zylindrisch ausgebildet sein.

Ferner umfaßt die Außenmutter ein Druckelement 25. Dieses weist einen mittleren ersten zylindrischen Abschnitt 26 auf, dessen Außendurchmesser im wesentlichen gleich dem Innendurchmesser des zylindrischen Wandabschnittes 22 ist. Er ist so bemessen, daß das Druckelement durch Einführen dieses Abschnittes in das Innere des Mantels durch Reibungskraft im zylindrischen Wandabschnitt 22 gehalten wird. Auf der dem Boden 19 der Hülse 17 zugewandten Seite ist koaxial zum ersten zylindrischen Abschnitt ein zweiter zylindrischer Abschnitt 27 vorgesehen. Der Durchmesser dieses Ansatzes entspricht im wesentlichen dem Durchmesser der Bohrung 16 und ist so bemessen, daß dieser Zylinderabschnitt in der Bohrung 16 aufgrund der Reibungskraft in dieser gehalten wird. Auf der dem Boden 19 abgewandten Seite des ersten zylindrischen Abschnittes 26 ist ein sich konvex erstreckender Abschnitt 28 vorgesehen, der annähernd kegelabschnittsförmig ausgebildet ist und mit seinem kleinen Durchmesser dem Abschnitt 26 zugewandt ist. Der kleine Durchmesser dieses nahezu kegelstumpfförmigen Abschnittes 28 ist nahezu gleich dem kleinen Innendurchmesser des Wandabschnittes 23, und der große Durchmesser des kegelförmigen Abschnittes 28 ist nahezu gleich dem großen Innendurchmesser des Wandabschnittes 23. Der Winkel des Abschnittes 28 ist im wesentlichen gleich dem Winkel des Wandabschnittes 23. An den Abschnitt 28 schließt sich ein dritter zylindrischer Abschnitt 29 an.

Wie am besten aus Fig. 4 ersichtlich ist, ist das Druckelement 25 in das Innere der Hülse 17 so weit eingeschoben, bis der konvergente Abschnitt 28 im Inneren der Hülse anliegt. Die Abmessungen des Druckelementes in axialer Richtung sind dabei wie folgt bestimmt: Die axiale Länge des ersten zylindrischen Abschnittes 26 einschließlich eines in Fig. 4 angedeuteten abgeschrägten Randes ist so bestimmt, daß bei Anliegen des Abschnittes 28 an dem Inneren der Hülse zwischen dem Boden 19 und der diesem zugewandten Oberfläche des ersten zylindrischen Abschnittes 26 ein spaltförmiger Abstand 32 von vorzugsweise weniger als einem Millimeter verbleibt. Die Länge des zweiten zylindrischen Abschnittes 27 ist gleich der Länge der aus der Bohrung 16 und der Bohrung 20 zusammengesetzten Ausnehmung. Die axiale Länge des dritten Abschnittes 29 ist so bemessen, daß dieser in der in Fig. 4 gezeigten Weise in der hier beschriebenen Ausgangsposition um ein Maß über den unteren Rand 33 der Hülse 17 hervorsteht, welches größer oder wenigstens gleich dem Maß des spaltförmigen Abstandes 32 ist.

Im Betrieb sind zunächst das Schraubenelement 1, das Aufnahmeteil 5 und die Druckscheibe in der an sich bekannten Weise zusammengesetzt, wie dies am besten aus Fig. 2 ersichtlich ist. Dann wird ein mit der Knochenschraube zu verbindender Stab 34 eingesetzt. Die Außenmutter 13 ist in dem in Fig. 4 ersichtlichen Zustand vormontiert, das heißt, das Druckelement 25 ist in die Hülse 17 eingesetzt und zusammen mit dieser dadurch mit dem Deckelteil 15 verbunden. Die so vormontierte Mutter wird nun auf das Außengewinde des Aufnahmeteiles 5 aufgeschraubt. Dabei liegt der Stab 34 einerseits auf der Druckscheibe 11 auf. Andererseits wird er durch das beim Aufschrauben mit dem Stab in Kontakt gelangende Druckelement 22 mit dem Druck beaufschlagt, so daß die Außenmutter 13 die Endposition nahezu erreicht hat. Beim weiteren Festziehen der Außenmutter 13 in die gewünschte Endposition wird das Druckelement 25 in der aus Fig. 5 ersichtlichen Weise bis zum Boden 19 geschoben, was gleichzeitig zur Folge hat, daß der Abschnitt 28 so auf die Innenwandung der Hülse 17 einwirkt, daß diese in der in Fig. 5 gezeigten Weise ein wenig nach außen gedrückt wird. Dadurch wird erreicht, daß der Mantel 18 mit seiner Außenfläche wiederum eine Kraft auf die freien Schenkel 30, 31 in dem Bereich des Außengewindes 10 in den das Innengewinde aufweisenden Mantel 18 hineindrücken.

Wie Fig. 3 am besten erkennen läßt, weist der Mantel 18 eine Mehrzahl von sich über die gesamte Mantellänge erstreckende. Schlitze 35 auf, die in Umfangsrichtung zueinander beabstandet sind und die den oben beschriebenen Vorgang des Aufweitens des Mantels 18 beim Hineindrücken des Druckelementes 25 erleichtern.

Durch das Vorsehen des an den konvexen Wandabschnitt 23 anschließenden Wandabschnittes 24 wird eine Aufspreizung im Wandabschnitt 24 erreicht, die dazu führt, daß der Außendurchmesser ohne Gegendruck von außen auf die Hülse im Bereich des unteren Randes 33 größer ist als der Außendurchmesser der Hülse im Bereich des konvexen Wandabschnittes 23. Das ermöglicht eine größere Toleranz für die relative Bemessung vom Außendurchmesser der Hülse 17 relativ zum Innendurchmesser der zweiten Bohrung 8, da auch dann, wenn der Innendurchmesser der zweiten Bohrung 8 etwas größer ist als der Außendurchmesser der Hülse 17, noch ein Druck auf die Schenkel 30, 31 ausgeübt wird, der für die Verriegelung ausreichend ist. Durch diese Verlängerung der Hülse und das Aufteilen derselben durch die Schlitze 35 in einzelne Wandabschnitte wirken diese wie eine Art Biegebalken, so daß die Verriegelung auch elastischer erfolgt.

In dem oben beschriebenen Ausführungsbeispiel handelt es sich um eine sogenannte Polyaxialschraube, bei der das Schraubenelement 1 und das Aufnahmeteil 5 winkelmäßig relativ zueinander bewegbar sind. In einer abgewandelten Ausführungsform sind das Schraubenelement 1 und ein den Stab 34 aufnehmendes Aufnahmeteil einstückig miteinander ausgebildet, etwa derart, daß das in der in Fig. 2 gezeigten Weise das Aufnahmeteil 5, der Kopf 3 und die Druckscheibe 11 einstückig ausgebildet sind. Die Außenmutter 13 weist in diesem Fall die identische oben beschriebene Form auf. In der Betriebsweise erfolgt die Arretierung der Außenmutter 13 durch Einwirken der Kraft vom Stab 34 auf das Druckelement 25 in der oben beschriebenen Weise, so daß die gleiche Verriegelung erzielt wird.

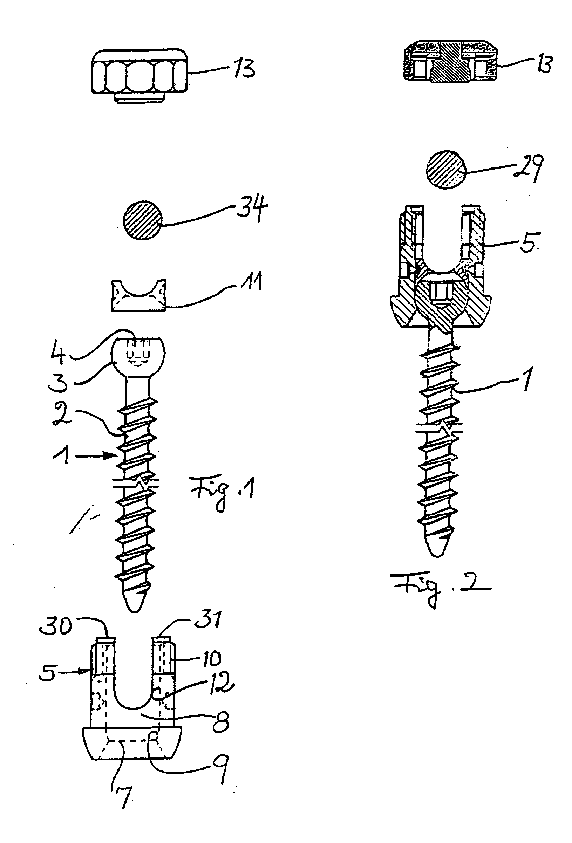

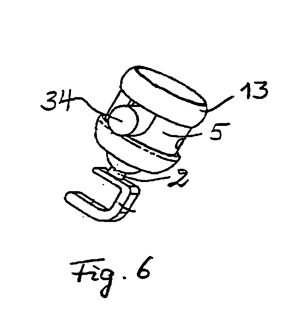

Die in Fig. 6 gezeigte Ausführungsform unterscheidet sich von den anhand der Fig. 1 bis 5 beschriebenen Ausführungsformen nur dadurch, daß anstelle des Schraubenelementes 1 mit dem Gewindeabschnitt 2 zwei ein Schaft 36 mit einem Haken 37 vorgesehen ist. Alle übrigen Elemente stimmen mit den vorher beschriebenen Elementen vollständig überein. Bei dem Haken handelt es sich um einen solche, der insbesondere an den hinteren Knochenvorsprüngen der Wirbelsäule eingehängt wird, z.B. an den Laminabögen, in Querfortsätzen oder in Zwischenwirbelräumen. Die Form und Abmessungen sind aus dem allgemeinen Stand der Technik bekannt.

In den oben beschriebenen Ausführungsbeispielen weist die Hülse 17 einen Boden 19 auf. In einer abgewandelten Ausführungsform weist die Hülse 17 keinen Boden auf. Die Hülse liegt dann mit ihrem Mantel 18 unmittelbar an der Innenseite 36 des Deckelteiles 15, und der Spalt 32 wird entsprechend zwischen der der Innenseite zugewandten Oberfläche des ersten zylindrischen Abschnittes 26 und der Innenseite 36 gebildet. Die Länge des zweiten zylindrischen Abschnittes 27 ist dann gleich der Länge der Bohrung 16.

## Patentansprüche

1. Fixierelement mit einem Schaft und einem kopfseitigen zylindrischen Aufnahmeteil (5) für die Aufnahme eines mit dem Fixierelement zu verbindenden Stabes (29), wobei das Aufnahmeteil eine offene Bohrung (8) besitzt und einen im wesentlichen U-förmigen Querschnitt mit zwei freien, ein Außengewinde (10) besitzenden Schenkeln (30, 31) aufweist, und mit einer auf das Außengewinde (10) aufschraubbaren Außenmutter (13), mit einem das Innengewinde aufweisenden Rand (14) und einem Deckelteil (15),
**dadurch gekennzeichnet, daß** die Außenmutter (13) im Inneren ein ein vorgegebenes Innenmaß aufweisendes hülsenförmiges Element (17) mit einem Mantel (18) mit einem dem Deckelteil (15) zugewandten ersten Rand und einem diesem abwandten zweiten Rand (33), einen inneren ersten Wandabschnitt (22) mit einem ersten Durchmesser und einem in Richtung zum zweiten Rand (33) hin folgenden inneren in Richtung zum zweiten Rand (33) konvex verlaufenden zweiten Wandabschnitt (23) und einen daran anschließenden dritten Wandabschnitt (24), der Außendurchmesser des Mantels (18) nahezu gleich oder wenig kleiner als der Durchmesser der Bohrung (8) ist, und ein darin angeordnetes Druckelement (25) aufweist, wobei das Druckelement (25) einen Abschnitt (28) aufweist, dessen Außenmaß größer als der erste Durchmesser ist und der beim Ausüben von Druck auf den aufnehmenden Stab (29) eine Aufweitung des Mantels (18) bewirkt.

2. Fixierelement nach Anspruch 1, **dadurch gekennzeichnet, daß** das hülsenförmige Element (17) auf der dem Deckelteil (15) zugewandten Seite einen zylindrischen Wandabschnitt (22) vorgegebener Länge und das Druckelement (25) einen damit in Eingriff befindlichen ersten zylindrischen Abschnitt (26), aufweisen.

3. Fixierelement nach Anspruch 1 oder 2, **dadurch gekennzeichnet, daß** der Mantel (18) zu dem unteren Rand hin geschlitzt ist.

4. Fixierelement nach einem der Ansprüche 1 bis 3, **dadurch gekennzeichnet, daß** das Druckelement (25) angrenzend an den Abschnitt (28) auf dessen dem Deckelteil (15) abgewandten Seite einen dritten Abschnitt (29') aufweist.

5. Fixierelement nach Anspruch 4, **dadurch gekennzeichnet, daß** der dritte Abschnitt (29') zylindrisch ausgebildet ist.

6. Fixierelement nach einem der Ansprüche 1 bis 5, **dadurch gekennzeichnet, daß** der Schaft einen Gewindeabschnitt (2) aufweist.

## Claims

1. Fixing element (2) with a shank and a cylindrical receiving part (5) on the head-side for receiving a rod (29) to be connected to the fixing element, wherein the receiving part has an open bore (8) and a substantially U-shaped cross-section with two exposed legs (30, 31) possessing an outer thread (10), and with an outer nut (13), which can be screwed on to the outer thread (10), with an edge (14) comprising the inner thread and a cover part (15), **characterised in that** the outer nut (13) has on the inside a sleeve-shaped element (17), having a predetermined inner measurement, with a casing (18) with a first edge facing the cover part (15) and a second edge (33) facing away from it, an inner first wall section (22) with a first diameter and an inner second wall section (23), following in the direction of the second edge (33), running convexly in the direction of the second edge (33), and a third wall section (24) adjacent to it, the outer diameter of the casing (18) is almost identical to or slightly smaller than the diameter of the bore (8) and a pressure element (25) arranged therein, wherein the pressure element (25) has a section (28), the outer measurement of which is larger than the first diameter and which effects a widening of the casing (18) when pressure is exerted on the receiving rod (29).

2. Fixing element according to Claim 1, **characterised in that** the sleeve-shaped element (17) has a cylindrical wall section (22) of predetermined length on the side facing the cover part (15) and the element (22) has a first cylindrical section (26) which is in engagement with it.

3. Fixing element according to Claim 1 or 2, **characterised in that** the casing (18) is slit towards the lower edge.

4. Fixing element according to one of Claims 1 to 3, **characterised in that** the pressure element (25) has a third section (29') bordering on the section (28) on its side facing away from the cover part (15).

5. Fixing element according to Claim 4, **characterised in that** the third section (29') is constructed as cylindrical.

6. Fixing element according to one of Claims 1 to 5, **characterised in that** the shank has a thread section (2).

## Revendications

1. Élément de fixation avec un corps et avec du côté de la tête une partie de logement (5) cylindrique destinée à recevoir une tige (29) à assembler avec l'élément de fixation, la partie de logement possédant un alésage (8) ouvert et une section sensiblement en forme de U avec deux bras (30, 31) libres possédant un filetage extérieur (10), et avec un écrou extérieur (13) pouvant être vissé sur le filetage extérieur (10), avec un bord (14) orienté vers le filetage intérieur et une partie formant capuchon (15), **caractérisé en ce que** l'écrou extérieur (13) présente à l'intérieur un élément en forme de douille (17) ayant des dimensions intérieures prédéterminées avec une enveloppe (18) avec un premier bord orienté vers la partie formant capuchon (15) et un second bord (33) orienté à l'opposé de celui-ci, une première partie de paroi intérieure (22) avec un premier diamètre et une seconde partie de paroi intérieure (23) suivant en direction du second bord (33) et convexe en direction du second bord (33) et une troisième partie de paroi (24) faisant suite à celle-ci, le diamètre extérieur de l'enveloppe (18) étant presque égal ou légèrement inférieur au diamètre de l'alésage (8), et un élément de compression (25) disposé à l'intérieur, lequel élément de compression (25) possède une partie (28) dont la dimension extérieure est plus grande que le premier diamètre et qui réalise, lors de l'application de pression sur la tige à recevoir (29), un élargissement de l'enveloppe (18).

2. Élément de fixation selon la revendication 1, **caractérisé en ce que** l'élément en forme de douille (17) présente sur-son côté orienté vers la partie formant capuchon (15) une partie de paroi cylindrique (22) de longueur prédéterminée et l'élément de compression (25) présente une première partie cylindrique (26) qui se met en prise avec celle-ci.

3. Élément de fixation selon la revendication 1 ou 2, **caractérisé en ce que** l'enveloppe (18) est fendue en direction du bord inférieur.

4. Élément de fixation selon l'une des revendications 1 à 3, **caractérisé en ce que** l'élément de compression (25) présente à la limite de la partie (28) sur son côté orienté vers la partie formant capuchon (15) une troisième partie (29').

5. Élément de fixation selon la revendication 4, **caractérisé en ce que** la troisième partie (29') est de forme cylindrique.

6. Élément de fixation selon l'une ou l'ensemble des revendications 1 à 5, **caractérisé en ce que** le corps possède une partie filetée (2).
